# EUROPEAN PATENT APPLICATION

(11) **EP 4 786 465 A1**
(43) Date of publication of application: **05.08.2026**
(21) Application number: 24872880.0
(22) Date of filing: 25.09.2024
(51) Int. Cl.: C07D 403/04, A61K 31/4178, A61K 31/4725, A61P 35/00, C07D 401/04, C07D 487/04

(54) **NOVEL COMPOUND, AND PHARMACEUTICAL COMPOSITION FOR PREVENTION OR TREATMENT OF CANCER OR TUMORS COMPRISING SAME**

(30) Priority: 25.09.2023 KR 20230128347
(71) Applicant: Daewoong Pharmaceutical Co., Ltd., Hwaseong-si, Gyenoggi-do 18623 (KR)
(72) Inventor: KANG, Youngku, Yongin-si, Gyeonggi-do 17028 (KR); PARK, Sang Jun, Yongin-si, Gyeonggi-do 17028 (KR); LEE, Seul, Yongin-si, Gyeonggi-do 17028 (KR); KWON, Ahreum, Yongin-si, Gyeonggi-do 17028 (KR); YOO, Bora, Yongin-si, Gyeonggi-do 17028 (KR); KIM, Ji Duck, Yongin-si, Gyeonggi-do 17028 (KR); PARK, Joon Seok, Yongin-si, Gyeonggi-do 17028 (KR)
(74) Representative: Germain Maureau
(86) International application number: PCT/KR2024/014441
(87) International publication number: WO 2025/071172

(57) **Abstract**

The present disclosure relates to a compound represented by the following Chemical Formula 1, or a pharmaceutically acceptable salt thereof, and the compound according to the present disclosure can be used favorably for preventing or treating cancer or tumors. 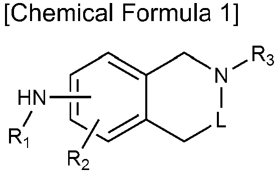 wherein in Chemical Formula 1, L and R₁ to R₄ are the same as defined in the detailed description.

## Description

### [TECHNICAL FIELD]

The present disclosure relates to a compound having a novel structure that can be used favorably for preventing or treating cancer or tumors.

### [BACKGROUND ART]

Hippo signaling pathway affects the size and number of cells constituting an organ, and affects organ development and cell growth. In addition, inappropriate regulation of Hippo signaling pathway has been associated with the development of several cancers (breast cancer, head and neck cancer, colon cancer, ovarian cancer, liver cancer, brain cancer, prostate cancer, mesothelioma, sarcoma, etc.).

The Hippo signaling system is composed of several components such as NF2(neurofibromatosis type 2), Mst1/2(mammalian Ste20-like kinases 1 and 2), Lats1/2(large tumor suppressor 1/2), YAP/TAZ(yes-associated protein/WW domain-containing transcription regulator protein), and TEADs(transcriptional enhanced associate domains). Among these, when mutations occur in upstream signaling proteins such as NF2, MST1/2, and LATS1/2, which are tumor suppressors, they induce a persistent binding of YAP/TAZ and TEAD. This promotes transcription by YAP/TAZ and TEAD to induce gene expression related to cancer cell proliferation and tumor formation.

When phosphorylation of Lats1/2 by Mst1/2 is mediated through activation of NF2 which is a tumor suppressor gene, phosphorylation of YAP and TAZ existing in the cytoplasm are promoted. The phosphorylated YAP and TAZ undergoes a ubiquitination process and are then decomposed by proteasome. Therefore, when the Hippo signaling system is turned on, YAP and TAZ are turned off. In contrast, under conditions in which tumor suppressor genes are turned off, i.e. when Hippo signaling is turned off, YAP and TAZ are turned on and translocate to the nucleus, which binds to four proteins of the TEAD family (TEAD1/2/3/4) and induces the expression of target genes such as CTGF (connective tissue growth factor), CYR61(cysteine-rich angiogenic inducer 61), Gli2 (GLI family zinc finger 2), Birc2/5(Baculoviral IAP repeat containing 2/5), FGF(fibroblast growth factor). In this manner, genes turned on by the YAP/TAZ-transcription factor complex regulate cell growth, cell migration, and apoptosis.

Abnormalities in the Hippo signaling system have been found in several carcinomas, and several research results have been reported to develop anticancer drugs targeting YAP-TEAD. Furthermore, the relevance of the Hippo signaling system has been revealed in the process of acquiring resistance after application of existing approved anticancer drugs, and several studies are underway to demonstrate the potential of YAP-TEAD anticancer drugs as combination therapeutics to treat drug resistance. Therefore, there is a need to develop a small molecule inhibitor for treating cancer caused by dysregulation of the Hippo signaling system.

In this regard, the present inventors have studied medicines that can be used favorably for treating cancer in which the regulation of Hippo signaling is turned off, and as a result, found that the compound according to the present disclosure described hereinafter binds to the palmitate binding site that mediates TEAD palmitoylation, and also inhibits the in vitro growth of a Hippo pathway mutated cell line. Through this, it has been found that the YAP-TEAD inhibitor according to the present disclosure can be used favorably for treating cancer or tumors in which Hippo signaling is turned off, and completed the present disclosure.

### [DETAILED DESCRIPTION OF THE INVENTION]

### [Technical Problem]

It is an object of the present disclosure to provide a compound having a novel structure that can be used favorably for preventing or treating cancer or tumors.

It is another object of the present disclosure to provide a pharmaceutical composition for the prevention or treatment of cancer or tumors comprising the above compound.

### [Technical Solution]

In order to achieve the above object, provided herein is a compound represented by the following Chemical Formula 1, or a pharmaceutically acceptable salt thereof: 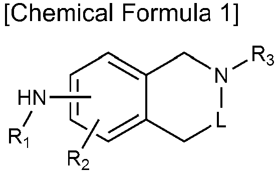
in Chemical Formula 1,
L is a single bond, or a C₁₋₃ linear alkylene,
R₁ is a C₆₋₁₀ aryl, or a 5- or 6-membered heterocycle containing 1 to 4 heteroatoms each independently selected from the group consisting of N, O and S,
   wherein the R₁ is unsubstituted, or substituted with 1 to 3 substituents each independently selected from the group consisting of halogen, a C₁₋₄ alkyl, a C₁₋₄ thioalkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, a C₃₋₆ cycloalkyl, amino, nitro, cyano, a (C₁₋₄ alkyl)amino, and a di(C₁₋₄ alkyl)amino,
R₂ is a C₆₋₁₀ aryl, or a 5- or 6-membered heterocycle containing 1 to 4 heteroatoms each independently selected from the group consisting of N, O and S,
   wherein the R₂ is unsubstituted, or substituted with halogen, a C₁₋₄ alkyl, a C₁₋₄ thioalkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, a C₃₋₆ cycloalkyl, amino, nitro, cyano, a (C₁₋₄ alkyl)amino, and a di(C₁₋₄ alkyl)amino, or is fused with a C₃₋₆ cycloalkyl ring,
R₃ is -CO-R₄, -SO₂-NH-R₄, or -CH₂-R₄, and
R₄ is hydrogen, a C₁₋₄ alkyl, a C₂₋₄ alkynyl, or cyano.

Preferably, L is a single bond, or methylene (-CH₂-).

Preferably, the Chemical Formula 1 is represented by any one of the following Chemical Formulas 1-1 to 1-4: 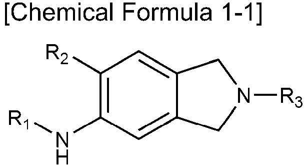 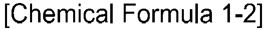 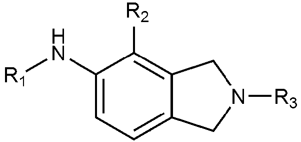 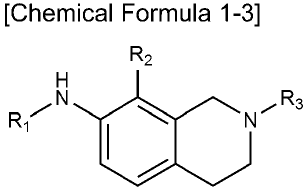 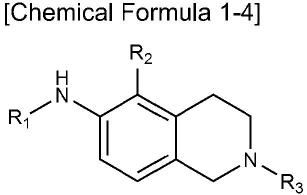
in Chemical Formulas 1-1 to 1-4,
R₁, R₂ and R₃ are as defined above.

Preferably, R₁ is phenyl, pyridinyl, pyrimidinyl, imidazolyl, pyrazolyl, triazolyl, tetrazolyl, oxadiazolyl, pyrrolidinyl, imidazolyl, pyrazolyl, oxazolyl, isoxazolyl, thiazolyl, or isothiazolyl,
wherein the R₁ is unsubstituted, or substituted with halogen, a C₁₋₄ alkyl, a C₁₋₄ thioalkyl, a C₁₋₄ haloalkyl, a C₁₋₄ alkoxy, a C₁₋₄ haloalkoxy, amino, nitro, cyano, a (C₁₋₄ alkyl)amino, or a di(C₁₋₄ alkyl)amino.

Preferably, R₁ is phenyl,
wherein the R₁ is unsubstituted, or substituted with 1 to 3 substituents each independently selected from the group consisting of fluoro, chloro, methyl and trifluoromethyl.

Preferably, R₂ is phenyl, pyridinyl, pyrimidinyl, imidazolyl, pyrazolyl, triazolyl, tetrazolyl, oxadiazolyl, pyrrolidinyl, imidazolyl, pyrazolyl, oxazolyl, isoxazolyl, thiazolyl, or isothiazolyl,
wherein the R₂ is unsubstituted, or substituted with halogen, a C₁₋₄ alkyl, a C₁₋₄ thioalkyl, a C₁₋₄ haloalkyl, a C₁₋₄ alkoxy, a C₁₋₄ haloalkoxy, amino, nitro, cyano, a (C₁₋₄ alkyl)amino, or di(C₁₋₄ alkyl)amino, or is fused with a C₃₋₆ cycloalkyl ring.

Preferably, R₂ is imidazolyl, wherein the R₂ is substituted with methyl, or ethyl, or is fused with a cyclopentane ring.

Preferably, R₂ is imidazolyl substituted with methyl, and more preferably, R₂ is 1-methyl-1H-imidazol-4-yl.

Preferably, R₄ is hydrogen, methyl, ethenyl, or cyano.

Preferably, R₃ is -CO-CH=CH₂, -SO₂-NH-CH₃, or -CH₂-CN.

Representative examples of the compounds represented by Chemical Formula 1 are as follows:
1) 1-(5-(1-methyl-1H-imidazol-4-yl)-6-((4-(trifluoromethyl)phenyl)amino)isoindolin-2-yl)prop-2-en-1-one,
2) N-methyl-5-(1-methyl-1H-imidazol-4-yl)-6-((4-(trifluoromethyl)phenyl)amino)isoindoline-2-sulfonamide,
3) 1-(5-(1-methyl-1H-imidazol-4-yl)-6-((4-(trifluoromethyl)phenyl)amino)-3,4-dihydroisoquinolin-2(1H)-yl)prop-2-en-1-one,
4) N-methyl-5-(1-methyl-1H-imidazol-4-yl)-6-((4-(trifluoromethyl)phenyl)amino)-3,4-dihydroisoquinoline-2(1H)-sulfonamide,
5) 1-(4-(1-methyl-1H-imidazol-4-yl)-5-((4-(trifluoromethyl)phenyl)amino)isoindolin-2-yl)prop-2-en-1-one,
6) N-methyl-4-(1-methyl-1H-imidazol-4-yl)-5-((4-(trifluoromethyl)phenyl)amino)isoindoline-2-sulfonamide,
7) 1-(5-(6,7-dihydro-5H-pyrrolo[1,2-a]imidazol-2-yl)-6-((4-(trifluoromethyl)phenyl)amino)-3,4-dihydroisoquinolin-2(1H)-yl)prop-2-en-1-one,
8) 2-(5-(1-methyl-1H-imidazol-4-yl)-6-((4-(trifluoromethyl)phenyl)amino)isoindolin-2-yl)acetonitrile,
9) 1-(6-((3-fluoro-4-(trifluoromethyl)phenyl)amino)-5-(1-methyl-1H-imidazol-4-yl)-3,4-dihydroisoquinolin-2(1H)-yl)prop-2-en-1-one,
10) 1-(6-((4-fluorophenyl)amino)-5-(1-methyl-1H-imidazol-4-yl)-3,4-dihydroisoquinolin-2(1H)-yl)prop-2-en-1-one,
11) 1-(5-((4-fluorophenyl)amino)-6-(1-methyl-1H-imidazol-4-yl)isoindolin-2-yl)prop-2-en-1-one,
12) 1-(7-((4-fluorophenyl)amino)-8-(1-methyl-1H-imidazol-4-yl)-3,4-dihydroisoquinolin-2(1H)-yl)prop-2-en-1-one,
13) 1-(6-((3,5-difluorophenyl)amino)-5-(1-methyl-1H-imidazol-4-yl)-3,4-dihydroisoquinolin-2(1H)-yl)prop-2-en-1-one,
14) 1-(5-(1-methyl-1H-imidazol-4-yl)-6-((3,4,5-trifluorophenyl)amino)-3,4-dihydroisoquinolin-2(1H)-yl)prop-2-en-1-one,
15) 1-(6-((4-chlorophenyl)amino)-5-(1-methyl-1H-imidazol-4-yl)-3,4-dihydroisoquinolin-2(1H)-yl)prop-2-en-1-one,
16) 1-(6-((4-bromophenyl)amino)-5-(1-methyl-1H-imidazol-4-yl)-3,4-dihydroisoquinolin-2(1H)-yl)prop-2-en-1-one,
17) 1-(6-((3,5-difluoro-4-(trifluoromethyl)phenyl)amino)-5-(1-methyl-1H-imidazol-4-yl)-3,4-dihydroisoquinolin-2(1H)-yl)prop-2-en-1-one,
18) 1-(5-(1-methyl-1H-imidazol-4-yl)-6-((3,4,5-trifluorophenyl)amino)isoindolin-2-yl)prop-2-en-1-one,
19) 1-(5-((4-chlorophenyl)amino)-6-(1-methyl-1H-imidazol-4-yl)isoindolin-2-yl)prop-2-en-1-one,
20) 1-(5-(1-methyl-1H-imidazol-4-yl)-6-((3-(trifluoromethyl)phenyl)amino)-3,4-dihydroisoquinolin-2(1H)-yl)prop-2-en-1-one,
21) 1-(6-((3,5-dichlorophenyl)amino)-5-(1-methyl-1H-imidazol-4-yl)-3,4-dihydroisoquinolin-2(1H)-yl)prop-2-en-1-one,
22) 1-(6-((3-chloro-5-(trifluoromethyl)phenyl)amino)-5-(1-methyl-1H-imidazol-4-yl)-3,4-dihydroisoquinolin-2(1H)-yl)prop-2-en-1-one,
23) 1-(6-((3,5-bis(trifluoromethyl)phenyl)amino)-5-(1-methyl-1H-imidazol-4-yl)-3,4-dihydroisoquinolin-2(1H)-yl)prop-2-en-1-one, and
24) 1-(5-(1-ethyl-1H-imidazol-4-yl)-6-((4-(trifluoromethyl)phenyl)amino)-3,4-dihydroisoquinolin-2(1H)-yl)prop-2-en-1-one.

In addition, the compounds of the present disclosure may exist in the form of salts, especially pharmaceutically acceptable salts. As salts, salts commonly used in the art, such as acid addition salts formed by pharmaceutically acceptable free acids can be used without limitation. The term "pharmaceutically acceptable salt" as used herein refers to any organic or inorganic addition salt of the compound represented by Chemical Formula 1, whose concentration is relatively non-toxic and harmless to ae patient and activates effectively and whose side effects do not degrade the beneficial efficacy of the above compound.

As the free acid, an organic acid and an inorganic acid can be used. Examples of the inorganic acids include hydrochloric acid, phosphoric acid, sulfuric acid, nitric acid, tartaric acid and the like. Examples of the organic acids include methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycollic acid, gluconic acid, galacturonic acid, glutamic acid, glutaric acid, glucuronic acid, aspartic acid, ascorbic acid, carbonic acid, vanillic acid, hydroiodic acid and the like, but are not limited thereto.

In addition, a pharmaceutically acceptable metal salt can be obtained by a conventional method using a base. For example, a compound represented by Chemical Formula 1 is dissolved in an excessive amount of an alkali metal hydroxide or an alkaline earth metal hydroxide solution, the non-soluble salt is filtered, and then the filtrate is evaporated and dried to obtain a pharmaceutically acceptable metal salt. At this time, it is particularly preferable to prepare a sodium salt, a potassium salt or a calcium salt as the metal salt.

Further, a pharmaceutically unacceptable salt or solvate of the compound represented by Chemical Formula 1 may be used as an intermediate when preparing the compound represented by Chemical Formula 1, or the pharmaceutically acceptable salt or the solvate thereof.

Meanwhile, the compound represented by Chemical Formula 1 can be prepared as shown in the following Reaction Scheme 1.

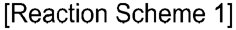

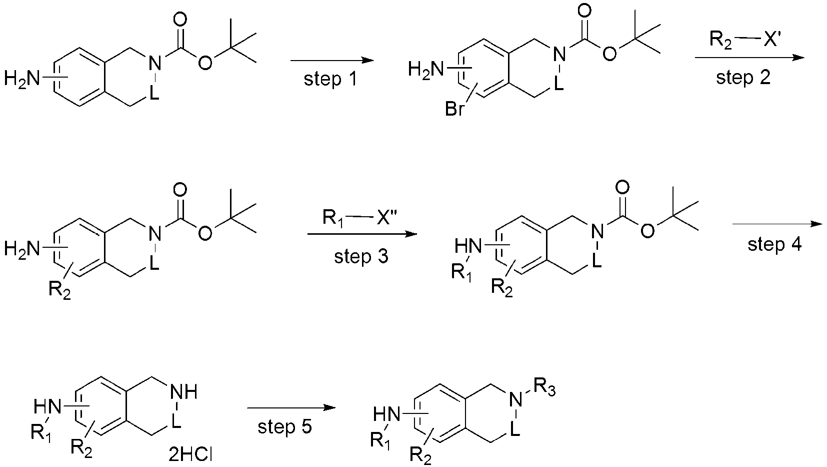

Step 1 is a bromination reaction, and step 2 is a Stille coupling reaction, wherein X' is a substituent for the Stille coupling reaction, and may be -SnBu₃, or - B(OH)₂ for the Suzuki reaction, or the like, but is not limited thereto.

Step 3 is a Chan-Lam reaction, wherein X'' is a substituent for the -B(OH)₂ coupling reaction, and may be -B(OH)₂, or a halogen for the Buchwald reaction, or the like, but is not limited thereto.

Step 4 is a reaction for removing a protecting group, which reaction may be carried out using an acid, and the type of the acid is not particularly limited.

Step 5 of the Reaction Scheme is a reaction between an amine group and a carbonyl group or a sulfonyl group according to R₃.

The preparation method may be more specifically described in the Examples provided hereinafter.

Further provided is a pharmaceutical composition comprising the compound represented by Chemical Formula 1, or a pharmaceutically acceptable salt thereof. Specifically, provided is a pharmaceutical composition for the prevention or treatment of cancer or tumors, comprising the compound represented by Chemical Formula 1, or a pharmaceutically acceptable salt thereof as an active ingredient.

The terms "cancer" or "tumor" refer to the presence, e.g., in a subject, of cells possessing characteristics typical of cancer-causing cells, such as uncontrolled proliferation, immortality, metastatic potential, rapid growth and proliferation rate, decreased cell death/apoptosis. Cancer cells often include solid tumors, such as a sarcomas or a carcinomas. The sarcomas includes alveolar rhabdomyosarcoma, alveolar soft part sarcoma, ameloblastoma, angiosarcoma, chondrosarcoma, chordoma, clear cell sarcoma, dedifferentiated liposarcoma, desmoid, connective tissue small round cell tumor, embryonal rhabdomyosarcoma, epithelioid fibrosarcoma, epithelioid hemangioendothelioma, epithelioid sarcoma, esthesioneuroblastoma, Ewing sarcoma, extrarenal rhabdoid tumor, extraskeletal myxoid chondrosarcoma, extrasketetal osteosarcoma, fibrosarcoma, giant cell tumor, hemangiopericytoma, infantile fibrosarcoma, inflammatory myofibroblastic tumor, Kaposi sarcoma, osteosarcoma, liposarcoma, liposarcoma of bone, malignant fibrous histiocytoma, malignant fibrous histiocytoma of bone, malignant mesenchymoma, malignant peripheral nerve sheath tumor, mesenchymal chondrosarcoma, myxofibrosarcoma; myxoid liposarcoma, myxoinflammatory fibroblastic sarcoma, osteosarcoma, parosteal osteosarcoma, periosteal osteosarcoma, pleomorphic liposarcoma, pleomorphic rhabdomyosarcoma, extraskeletal Ewing tumor, rhabdomyosarcoma, round cell liposarcoma, small cell osteosarcoma, solitary fibrous tumor, synovial sarcoma, and telangiectatic osteosarcoma. The carcinoma includes an adenocarcinoma, squamous cell carcinoma, adenosquamous carcinoma, anaplastic carcinoma, large cell carcinoma, small cell carcinoma, anal cancer, appendix cancer; bile duct cancer, bladder cancer, brain tumor, breast cancer, cervical cancer, colon cancer, cancer of Unknown Primary, esophageal cancer, eye cancer, primary fallopian tube cancer, digestive organ cancer, kidney cancer, liver cancer, lung cancer, medulloblastoma, malignant melanoma, oral cancer, ovarian cancer, pancreatic cancer, parathyroid cancer, penile tumor, pituitary tumor, prostate cancer, rectal cancer, skin cancer, stomach cancer, testicular cancer, throat cancer, thyroid cancer, uterine cancer, vaginal cancer, and vulvar cancer. In some embodiments, the carcinoma is breast cancer. In some embodiments, the cancer used in this application include may include uveal melanoma, mesothelioma, esophageal cancer, liver cancer, breast cancer, hepatocellular carcinoma, lung adenocarcinomas, glioma, colorectal cancer, colon cancer, stomach cancer, medulloblastoma, ovarian cancer, esophageal squamous cell carcinoma, sarcoma, Ewing sarcoma, head and neck cancer, prostate cancer, and meningioma.

Cancer also includes non-solid tumors, such as blood cancers, wherein the cancer cells are derived from bone marrow. The blood cancer includes leukemia, lymphoma, myeloma, non-Hodgkin lymphoma, Hodgkin lymphoma, T-cell malignancy, or B-cell malignancy. In particular, the T-cell malignancy includes mature T-cell lymphoma not elsewhere classified, anaplastic large cell lymphoma, angioimmunoblastic lymphoma, cutaneous T-cell lymphoma, adult T-cell leukemia, blastic natural killer cell lymphoma, platelet gamma-delta T-cell lymphoma, and lymphoblastic lymphoma. The B-cell malignancy includes chronic lymphocytic leukemia, small lymphocytic lymphoma, follicular lymphoma, diffuse large B-cell lymphoma, mantle cell lymphoma, Waldenström macroglobulinemia, head and neck tumor, nodal marginal zone B cell lymphoma, Burkitt's lymphoma, primary mediastinal B-cell lymphoma, immune large cell lymphoma, progenitor B-lymphoblastic lymphoma, prolymphocytic leukemia, lymphoplasmacytic lymphoma, nasal peripheral zone lymphoma, plasma cell myeloma, plasmacytoma, intravascular large B-cell lymphoma, primary effusion lymphoma, or lymphomatoid granulomatosis, but is not limited thereto.

In some embodiments, cancer includes recurrent or refractory cancer, wherein the cancer is a solid tumor. The recurrent or incurable cancer includes adenocarcinoma, squamous cell carcinoma, adenosquamous cell carcinoma, anaplastic carcinoma, large cell carcinoma, small cell carcinoma, biliary tract cancer, bladder cancer, brain tumor, breast cancer, cervical cancer, colorectal cancer, esophageal cancer, ophthalmic tumor, primary fallopian tube cancer, kidney cancer, liver cancer, lung cancer, medulloblastoma, malignant melanoma, oral cancer, ovarian cancer, pancreatic cancer, penile cancer, pituitary tumor, prostate cancer, rectal cancer, skin cancer, stomach cancer, testis cancer, throat cancer, thyroid cancer, uterine cancer, vaginal cancer, and vulvar cancer.

As used herein, the term "prevention" refers to any act to delay or inhibit occurrence, spread or recurrence of the above-mentioned diseases by administration of the composition of the present disclosure, and "treatment" refers to any act to improve or change the symptoms of the above diseases for the better by administration of the composition of the present disclosure.

The pharmaceutical composition according to the present disclosure can be formulated in types for oral or parenteral administrations according to a standard pharmaceutical practice. These formulations may contain additives such as pharmaceutically acceptable carrier, adjuvant or diluent in addition to the active ingredient.

Suitable carriers include, for example, physiological saline, polyethylene glycol, ethanol, vegetable oil, and isopropyl myristate and the like. Diluents include, for example, lactose, dextrose, sucrose, mannitol, sorbitol, cellulose and/or glycine and the like, but are not limited thereto. Further, the compounds of the present disclosure can be dissolved in oils, propylene glycol or other solvents commonly used in the preparation of injection solutions. Furthermore, the compounds of the present disclosure can be formulated in ointments or creams for topical application.

A preferred dose of the compound of the present disclosure may be varied according to the condition and weight of a patient, the severity of a disease, the type of a drug, and the route and duration of administration, but it may be suitably selected by those skilled in the art. In order to achieve the desirable effects, however, the compound of the present disclosure may be administrated daily at a dose of 0.0001 to 100 mg/kg (body weight), and preferably 0.001 to 100 mg/kg (body weight). The administration may be performed once a day or in divided doses each day through an oral or parenteral route.

Depending on the method of administration, the pharmaceutical composition may contain the compound of the present disclosure in an amount of 0.001 to 99% by weight, preferably 0.01 to 60% by weight.

The pharmaceutical composition according to the present disclosure may be administered to mammals such as a rat, a mouse, a domestic animal, a human, through various routes. The administration may be carried out through all possible methods, for example, oral, rectal, intravenous, intramuscular, subcutaneous, intra-endometrial, intracerebroventricular injection.

### [Advantageous Effects]

A compound represented by Chemical Formula 1 of the present disclosure, or a pharmaceutically acceptable salt thereof can be used favorably for preventing or treating cancer or tumors.

### [BRIEF DESCRIPTION OF THE DRAWINGS

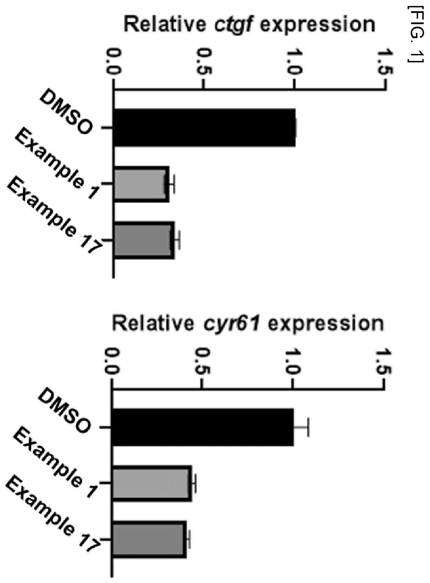

FIG. 1 shows the results of Experimental Example 3, and shows the expression regulatory properties of two TEAD target genes in Examples 1 and 17 using real-time PCR.

### [DETAILED DESCRIPTION OF THE EMBODIMENTS

Hereinafter, preferred examples are presented to assist in the understanding of the present disclosure. However, the following examples are for illustrative purposes only, and should not be construed as limiting the scope of the present disclosure to these examples.

### Example 1: Preparation of 1-(5-(1-methyl-1H-imidazol4-yl)-6-((4-(trifluoromethyl)phenyl)amino)isoindolin-2-yl)prop-2-en-1-one

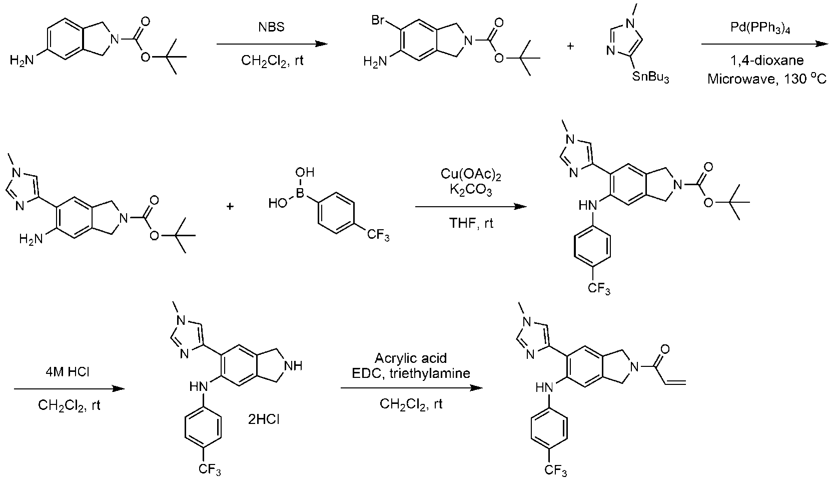

### (Step 1)

tert-Butyl 5-aminoisoindoline-2-carboxylate (8.54 mmol, 2.00 g, 1.0 eq) was dissolved in dichloromethane (43 mL), and then stirred at 0°C for 30 minutes. N-bromosuccinimide (8.54 mmol, 1.52 g, 1.0 eq) was slowly added thereto, and then reacted at room temperature for 4 hours. After the reaction was completed, the reaction mixture was extracted with dichloromethane and 10% potassium carbonate aqueous solution, concentrated, and the product was purified by a column chromatography to give tert-butyl 5-amino-6-bromoisoindoline-2-carboxylate (0.98 g, yield: 37%).

### (Step 2)

In a sealed tube, tert-butyl 5-amino-6-bromoisoindolin-2-carboxylate (1.47 mmol, 0.46 g, 1.0 eq) was dissolved in 1,4-dioxane (2.9 mL). 1-Methyl-4-(tributylstannyl)-1H-imidazole (1.76 mmol, 0.65 g, 1.2 eq) and Pd(PPh₃)₄ (0.15 mmol, 0.17 g, 0.1 eq) were sequentially added thereto, and then reacted in a microwave reactor at 130°C for 2 hours. After the reaction was completed, palladium was removed, and then 1,4-dioxane was concentrated and the product was purified by a column chromatography to give tert-butyl 5-amino-6-(1-methyl-1H-imidazol-4-yl)isoindoline-2-carboxylate (0.37 g, yield: 80%).

### (Step 3)

tert-Butyl 5-amino-6-(1-methyl-1H-imidazol-4-yl)isoindoline-2-carboxylate (1.49 mmol, 0.47 g, 1.0 eq), copper acetate (1.79 mmol, 0.33 g, 1.2 eq), and potassium carbonate (4.48 mmol, 0.62 g, 3.0 eq) were dissolved in tetrahydrofuran (7.5 mL), and then stirred at 0°C for 30 minutes. (4-(Trifluoromethyl)phenyl)boronic acid (1.75 mmol, 0.33 g, 1.1 eq) was slowly added thereto, and then reacted overnight at room temperature. After the reaction was completed, copper was removed, tetrahydrofuran was concentrated and the product was purified by a column chromatography to give tert-butyl 5-(1-methyl-1H-imidazol-4-yl)-6-((4-(trifluoromethyl)phenyl)amino)isoindoline-2-carboxylate (0.23 g, yield: 34%).

### (Step 4)

tert-Butyl 5-(1-methyl-1H-imidazol-4-yl)-6-((4-(trifluoromethyl)phenyl)amino)isoindoline-2-carboxylate(0.20 mmol, 0.091 g, 1.0 eq), dichloromethane (2.0 mL), and 4M hydrochloride 1,4-dioxane(0.50 mL) were sequentially added. The reactants were allowed to react overnight at room temperature. After the reaction was completed, the solidified 6-(1-methyl-1H-imidazol-4-yl)-N-(4-(trifluoromethyl)phenyl)isoindoline-5-amine dihydrochloride was filtered and used for the next reaction without purification.

### (Step 5)

6-(1-Methyl-1H-imidazol-4-yl)-N-(4-(trifluoromethyl)phenyl)isoindolin-5-amine dihydrochloride (0.29 mmol, 0.125 g, 1.0 eq), N-(3-dimethylaminopropyl)-N-ethylcarbodiimide hydrochloride (0.44 mmol, 0.083 g, 1.5 eq), and dichloromethane (2.9 mL) were sequentially added to a flask. Triethylamine (0.16 mL) and acrylic acid (0.35 mmol, 0.027 mL, 1.2 eq) were added thereto, and then reacted overnight at room temperature. After the reaction was completed, dichloromethane was removed and the product was purified by a column chromatography to give the title compound (25.0 mg, yield: 21%).

¹H NMR (500 MHz, CDCl₃) δ 10.10 and 9.96 (2s, 1H, rotamer), 7.53 (s, 1H), 7.52-7.47 (m, 2H, rotamer), 7.44 and 7.43 (2s, 1H, rotamer), 7.40 and 7.38 (2s, 1H, rotamer), 7.22 (d, J = 8.3 Hz, 2H), 7.20 and 7.16 (2s, 1H, rotamer), 6.62-6.54 (m, 1H, rotamer), 6.53-6.46 (m, 1H, rotamer), 5.80 (d, J = 10.0 Hz, 1H), 4.92 and 4.91 (2s, 2H, rotamer), 4.87 (s, 2H), 3.78 (s, 3H).

### Example 2: Preparation of N-methyl-5-(1-methyl-1H-imidazol-4-yl)-6-((4-(trifluoromethyl)phenyl)amino)isoindoline-2-sulfonamide

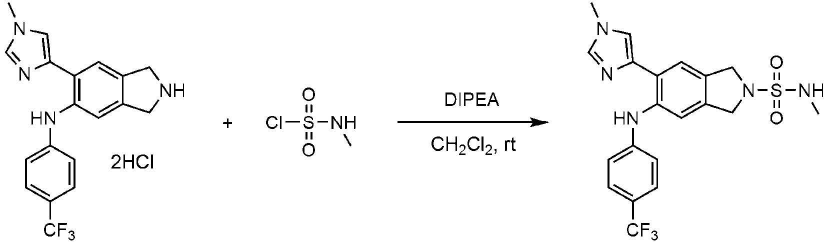

6-(1-Methyl-1H-imidazol-4-yl)-N-(4-(trifluoromethyl)phenyl)isoindolin-5-amine dihydrochloride (0.15 mmol, 0.060 g, 1.0 eq), and N,N-diisopropylethylamine (0.13 mL) were dissolved in dichloromethane (1.5 mL), and then stirred at 0□ for 10 minutes. Methylsulfomoyl chloride (0.23 mmol, 0.029 g, 1.5 eq) was slowly added thereto, and then reacted at room temperature for 4 hours. After the reaction was completed, dichloromethane was removed and the product was purified by column chromatography to give the title compound (5.1 mg, yield: 8%).

¹H NMR (500 MHz, CDCl₃) 10.01 (s, 1H), 7.53 (s, 1H), 7.49 (d, J = 8.5 Hz, 2H), 7.37 (s, 1H), 7.36 (s, 1H), 7.21 (d, J = 8.4 Hz, 2H), 7.17 (s, 1H), 4.68 (s, 2H), 4.67 (s, 2H), 4.26 (q, J = 5.4 Hz, 1H), 3.78 (s, 3H), 2.81 (d, J = 5.4 Hz, 3H).

### Example 3: Preparation of 1-(5-(1-methyl-1H-imidazol-4-yl)-6-((4-(trifluoromethyl)phenyl)amino)-3,4-dihydroisoquinolin-2(1H)-yl)prop-2-en-1-one

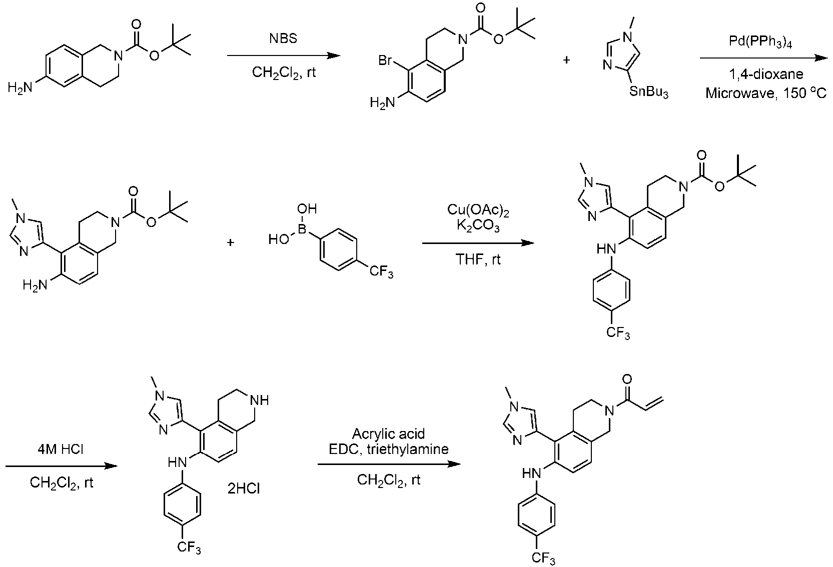

### (Step 1)

tert-Butyl 6-amino-3,4-dihydroisoquinoline-2(1H)-carboxylate (8.05 mmol, 2.00 g, 1.0 eq) was dissolved in dichloromethane (43 mL), and then stirred at 0°C for 30 minutes. N-bromosuccinimide (8.05 mmol, 1.43 g, 1.0 eq) was slowly added thereto, and then reacted at room temperature for 4 hours. After the reaction was completed, the reaction mixture was extracted with dichloromethane and 10% potassium carbonate aqueous solution, concentrated, and the product was purified by a column chromatography to give tert-butyl 6-amino-5-bromo-3,4-dihydroisoquinoline-2(1H)-carboxylate (1.82 g, yield: 69%).

### (Step 2)

In a sealed tube, tert-butyl 6-amino-5-bromo-3,4-dihydroisoquinoline-2(1H)-carboxylate (1.53 mmol, 0.50 g, 1.0 eq) was dissolved in 1,4-dioxane (3.1 mL). 1-Methyl-4-(tributylstannyl)-1H-imidazole (1.83 mmol, 0.68 g, 1.2 eq) and Pd(PPh₃)₄ (0.15 mmol, 0.18 g, 0.1 eq) were sequentially added thereto, and then reacted in a microwave reactor at 150°C for 1.5 hours. After the reaction was completed, palladium was removed, and then 1,4-dioxane was concentrated and the product was purified by a column chromatography to give tert-butyl 6-amino-5-(1-methyl-1H-imidazol-4-yl)-3,4-dihydroisoquinoline-2(1H)-carboxylate (0.49 g, yield: 98%).

### (Step 3)

6-Amino-5-(1-methyl-1H-imidazol-4-yl)-3,4-dihydroisoquinoline-2(1H)-carboxylate (1.56 mmol, 0.51 g, 1.0 eq), copper acetate (1.87 mmol, 0.34 g, 1.2 eq), and potassium carbonate (4.68 mmol, 0.65 g, 3.0 eq) were dissolved in tetrahydrofuran (7.8 mL), and then stirred at 0°C for 30 minutes. (4-(Trifluoromethyl)phenyl)boronic acid (1.71 mmol, 0.33 g, 1.1 eq) was slowly added thereto, and then reacted overnight at room temperature. After the reaction was completed, copper was removed, tetrahydrofuran was concentrated and the product was purified by a column chromatography to give tert-butyl 5-(1-methyl-1H-imidazol-4-yl)-6-((4-(trifluoromethyl)phenyl)amino)-3,4-dihydroisoquinoline-2(1H)-carboxylate (0.35 g, yield: 48%).

### (Step 4)

tert-Butyl 5-(1-methyl-1H-imidazol-4-yl)-6-((4-(trifluoromethyl)phenyl)amino)-3,4-dihydroisoquinoline-2(1H)-carboxylate (0.74 mmol, 0.35 g, 1.0 eq), dichloromethane (7.4 mL), and 4M hydrochloride 1,4-dioxane(1.85 mL) were sequentially added. The reactants were allowed to react overnight at room temperature. After the reaction was completed, the solidified 5-(1-methyl-1H-imidazol-4-yl)-N-(4-(trifluoromethyl)phenyl)-1,2,3,4-tetrahydroisoquinolin-6-amine dihydrochloride was filtered and used for the next reaction without purification.

### (Step 5)

5-(1-Methyl-1H-imidazol-4-yl)-N-(4-(trifluoromethyl)phenyl)-1,2,3,4-tetrahydroisoquinolin-6-amine dihydrochloride (0.74 mmol, 0.33 g, 1.0 eq), N-(3-dimethylaminopropyl)-N-ethylcarbodiimide hydrochloride (1.11 mmol, 0.21 g, 1.5 eq), and dichloromethane (7.4 mL) were sequentially added to a flask. Triethylamine (0.41 mL) and acrylic acid (0.89 mmol, 0.061 mL, 1.2 eq) were added thereto, and then reacted overnight at room temperature. After the reaction was completed, dichloromethane was removed and the product was purified by a column chromatography to give the title compound (114.8 mg, yield: 36%).

¹H NMR (500 MHz, CDCl₃) δ 8.52 and 8.21 (2s, 1H, rotamer), 7.60 (s, 1H), 7.43 (d, J = 8.5 Hz, 2H), 7.38 (d, J = 8.3 Hz, 1H), 7.16-7.01 (m, 3H, rotamer), 6.91 and 6.89 (2s, 1H, rotamer), 6.75-6.57 (m, 1H, rotamer), 6.38 (d, J = 16.7 Hz, 1H), 5.80-5.70 (m, 1H, rotamer), 4.80 and 4.76 (2s, 2H, rotamer), 3.84-3.66 (m, 5H, rotamer), 2.99-2.88 (m, 2H, rotamer).

### Example 4: Preparation of N-methyl-5-(1-methyl-1H-imidazol-4-yl)-6-((4-(trifluoromethyl)phenyl)amino)-3,4-dihydroisoquinoline-2(1H)-sulfonamide

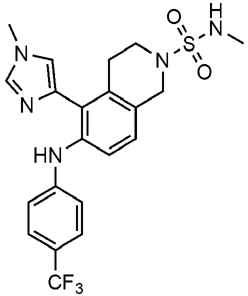

The title compound (3.2 mg, yield: 11%) was obtained in the same manner as in Example 2, except that 5-(1-methyl-1H-imidazol-4-yl)-N-(4-(trifluoromethyl)phenyl)-1,2,3,4-tetrahydroisoquinolin-6-amine dihydrochloride was used instead of 6-(1-methyl-1H-imidazol-4-yl)-N-(4-(trifluoromethyl)phenyl)isoindolin-5-amine dihydrochloride in Example 2.

¹H NMR (500 MHz, CDCl₃) δ 7.99 (s, 1H), 7.72 (s, 1H), 7.43 (d, J = 8.5 Hz, 2H), 7.35 (d, J = 8.4 Hz, 1H), 7.07-6.99 (m, 3H), 6.93 (s, 1H), 4.46 (s, 2H), 4.31 (d, J = 5.0 Hz, 1H), 3.79 (s, 3H), 3.48 (t, J = 5.7 Hz, 2H), 2.92 (t, J = 5.6 Hz, 2H), 2.77 (d, J = 5.2 Hz, 3H).

### Example 5: Preparation of 1-(4-(1-methyl-1H-imidazol-4-yl)-5-((4-(trifluoromethyl)phenyl)amino)isoindolin-2-yl)prop-2-en-1-one

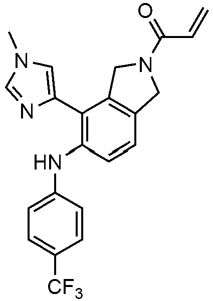

The title compound (9.1 mg, yield: 19%) was obtained in the same manner as in Example 1, except that tert-butyl 5-amino-4-bromoisoindolin-2-carboxylate was used instead of tert-butyl 5-amino-6-bromoisoindolin-2-carboxylate in step 2 of Example 1.

¹H NMR (500 MHz, DMSO) δ 9.89 and 9.60 (2s, 1H, rotamer), 7.85 and 7.83 (2s, 1H, rotamer), 7.57-7.43 (m, 3H, rotamer), 7.40-7.35 (m, 1H, rotamer), 7.27-7.19 (m, 1H, rotamer), 7.08-6.99 (m, 2H, rotamer), 6.87-6.67 (m, 1H, rotamer), 6.29-6.21 (m, 1H, rotamer), 5.81-5.74 (m, 1H, rotamer), 5.12 and 4.97 (2s, 2H, rotamer), 4.91 and 4.73 (2s, 2H, rotamer), 3.73 and 3.72 (2s, 3H, rotamer).

### Example 6: Preparation of N-methyl-4-(1-methyl-1H-imidazol-4-yl)-5-((4-(trifluoromethyl)phenyl)amino)isoindoline-2-sulfonamide

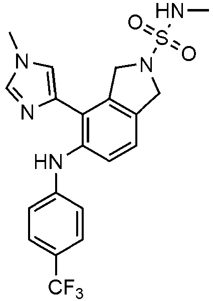

The title compound (9.6 mg, yield: 20%) was obtained in the same manner as in Example 2, except that 4-(1-methyl-1H-imidazol-4-yl)-N-(4-(trifluoromethyl)phenyl)isoindolin-5-amine dihydrochloride was used instead of 6-(1-methyl-1H-imidazol-4-yl)-N-(4-(trifluoromethyl)phenyl)isoindolin-5-amine dihydrochloride in Example 2.

¹H NMR (500 MHz, DMSO) δ 9.39 (s, 1H), 7.81 (s, 1H), 7.47 (d, J = 8.6 Hz, 2H), 7.40 (s, 1H), 7.34 (d, J = 8.1 Hz, 1H), 7.21 (d, J = 8.0 Hz, 1H), 6.99 (d, J = 8.5 Hz, 2H), 4.75 (s, 2H), 4.57 (s, 2H), 3.70 (s, 3H), 2.56 (d, J = 5.0 Hz, 3H), 2.00 (s, 1H).

### Example 7: Preparation of 1-(5-(6,7-dihydro-5H-pyrrolo[1,2-a]imidazol-2-yl)-6-((4-(trifluoromethyl)phenyl)amino)-3,4-dihydroisoquinolin-2(1H)-yl)prop-2-en-1-one

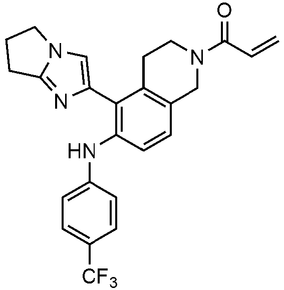

The title compound (8.1 mg, yield: 30%) was obtained in the same manner as in Example 1, except that 2-(tributylstannyl)-6,7-dihydro-5H-pyrrolo[1,2-a]imidazole was used instead of 1-methyl-4-(tributylstannyl)-1H-imidazole in step 2 of Example 1.

¹H NMR (500 MHz, CDCl₃) δ 8.66 and 8.39 (2s, 1H, rotamer), 7.43 (d, J = 8.5 Hz, 2H), 7.38 (d, J = 8.3 Hz, 1H), 7.13-7.00 (m, 3H, rotamer), 6.88 and 6.87 (2s, 1H, rotamer), 6.75-6.58 (m, 1H, rotamer), 6.38 (d, J = 16.8 Hz, 1H), 5.80-5.70 (m, 1H, rotamer), 4.79 and 4.75 (2s, 2H, rotamer), 4.07 (brs, 2H), 3.83-3.64 (m, 2H, rotamer), 3.04-2.90 (m, 4H, rotamer), 2.68 (qui, J = 7.5 Hz, 2H).

### Example 8: Preparation of 2-(5-(1-methyl-1H-imidazol-4-yl)-6-((4-(trifluoromethyl)phenyl)amino)isoindolin-2-yl)acetonitrile

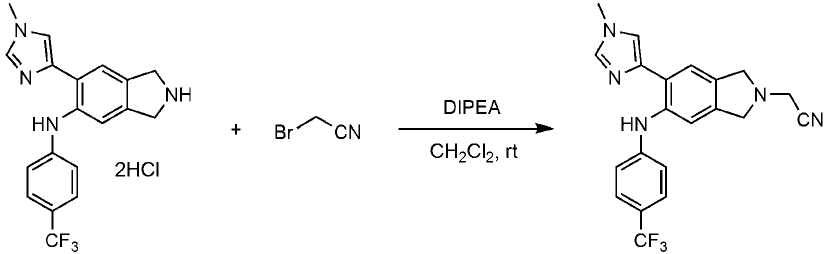

6-(1-Methyl-1H-imidazol-4-yl)-N-(4-(trifluoromethyl)phenyl)isoindolin-5-amine dihydrochloride (0.058 mmol, 0.025 g, 1.0 eq) and N,N-diisopropylethylamine (0.050 mL) were dissolved in dichloromethane (0.58 mL). 2-Bromoacetonitrile (0.087 mmol, 0.010 g, 1.5 eq) was slowly added thereto, and then reacted overnight at room temperature. After the reaction was completed, dichloromethane was removed and the product was purified by a column chromatography to give the title compound (22.6 mg, yield: 98%).

¹H NMR (500 MHz, CDCl₃) δ 9.97 (s, 1H), 7.50 (s, 1H), 7.48 (d, J = 8.5 Hz, 2H), 7.37 (s, 1H), 7.36 (s, 1H), 7.20 (d, J = 8.5 Hz, 2H), 7.14 (s, 1H), 4.09 (s, 4H), 3.84 (s, 2H), 3.73 (s, 3H).

### Example 9: Preparation of 1-(6-((3-fluoro-4-(trifluoromethyl)phenyl)amino)-5-(1-methyl-1H-imidazol-4-yl)-3,4-dihydroisoquinolin-2(1H)-yl)prop-2-en-1-one

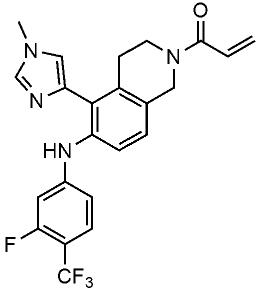

The title compound (9.0 mg, yield: 28%) was obtained in the same manner as in Example 3, except that (3-fluoro-4-(trifluoromethyl)phenyl)boronic acid was used instead of (4-(trifluoromethyl)phenyl)boronic acid in step 3 of Example 3.

¹H NMR (500 MHz, CDCl₃) δ 8.80 and 8.48 (2s, 1H, rotamer), 7.60 (s, 1H), 7.44-7.31 (m, 2H, rotamer), 7.20-7.03 (m, 1H, rotamer), 6.92 (s, 1H), 6.81-6.59 (m, 3H, rotamer), 6.38 (d, J = 16.7 Hz, 1H), 5.83-5.70 (m, 1H, rotamer), 4.82 and 4.77 (2s, 2H, rotamer), 3.89-3.63 (m, 5H, rotamer), 3.02-2.84 (m, 2H, rotamer).

### Example 10: Preparation of 1-(6-((4-fluorophenyl)amino)-5-(1-methyl-1H-imidazol-4-yl)-3,4-dihydroisoquinolin-2(1H)-yl)prop-2-en-1-one

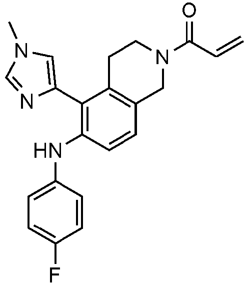

The title compound (4.3 mg, yield: 48%) was obtained in the same manner as in Example 3, except that (4-fluorophenyl)boronic acid was used instead of (4-(trifluoromethyl)phenyl)boronic acid in step 3 of Example 3.

¹H NMR (500 MHz, CDCl₃) 7.90 and 7.61 (2s, 2H, rotamer), 7.12-7.00 (m, 3H, rotamer), 6.96 (t, J = 8.2 Hz, 2H), 6.86-6.77 (m, 2H, rotamer), 6.74-6.58 (m, 1H, rotamer), 6.37 (d, J = 16.8 Hz, 1H), 5.79-5.69 (m, 1H, rotamer), 4.76 and 4.72 (2s, 2H, rotamer), 3.91-3.61 (m, 5H, rotamer), 2.97-2.89 (m, 2H, rotamer).

### Example 11: Preparation of 1-(5-((4-fluorophenyl)amino)-6-(1-methyl-1H-imidazol-4-yl)isoindolin-2-yl)prop-2-en-1-one

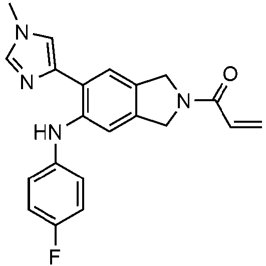

The title compound (4.0 mg, yield: 60%) was obtained in the same manner as in Example 1, except that (4-fluorophenyl)boronic acid was used instead of (4-(trifluoromethyl)phenyl)boronic acid in step 3 of Example 1.

¹H NMR (500 MHz, CDCl₃) δ 9.68 and 9.55 (2s, 1H, rotamer), 7.54 and 7.53 (2s, 1H, rotamer), 7.39 and 7.35 (2s, 1H, rotamer), 7.25-7.16 (m, 3H, rotamer), 7.14 and 7.10 (2s, 1H, rotamer), 7.06-6.96 (m, 2H, rotamer), 6.64-6.45 (m, 2H, rotamer), 5.82-5.73 (m, 1H, rotamer), 4.89 and 4.86 (2s, 2H, rotamer), 4.85 and 4.81 (2s, 2H, rotamer), 3.79 (s, 3H).

### Example 12: Preparation of 1-(7-((4-fluorophenyl)amino)-8-(1-methyl-1H-imidazol-4-yl)-3,4-dihydroisoquinolin-2(1H)-yl)prop-2-en-1-one

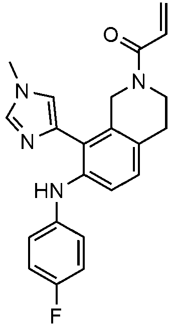

The title compound (3.0 mg, yield: 14%) was obtained in the same manner as in Example 3, except that tert-butyl 7-amino-3,4-dihydroisoquinoline-2(1H)-carboxylate was used instead of 6-amino-3,4-dihydroisoquinoline-2(1H)-carboxylate in step 1 of Example 3, and (4-fluorophenyl)boronic acid was used instead of (4-(trifluoromethyl)phenyl)boronic acid in step 3 of Example 3.

¹H NMR (500 MHz, CDCl₃) δ 7.62 (s, 1H), 7.12 (d, J = 8.0 Hz, 1H), 7.08-6.98 (m, 4H, rotamer), 6.97-6.88 (m, 3H, rotamer), 6.64 (dd, J = 16.7, 10.5 Hz, 1H), 6.33 (d, J = 15.8 Hz, 1H), 5.72 (d, J = 10.4 Hz, 1H), 4.78 and 4.65 (2s, 2H, rotamer), 3.86-3.72 (m, 5H, rotamer), 2.99-2.82 (m, 2H, rotamer).

### Example 13: Preparation of 1-(6-((3,5-difluorophenyl)amino)-5-(1-methyl-1H-imidazol-4-yl)-3,4-dihydroisoquinolin-2(1H)-yl)prop-2-en-1-one

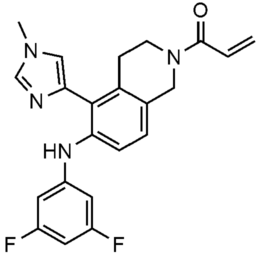

The title compound (7.0 mg, yield: 46%) was obtained in the same manner as in Example 3, except that (3,5-difluorophenyl)boronic acid was used instead of (4-(trifluoromethyl)phenyl)boronic acid in step 3 of Example 3.

¹H NMR (500 MHz, CDCl₃) δ 8.55 and 8.23 (2s, 1H, rotamer), 7.61 (s, 1H), 7.36 (d, J = 8.3 Hz, 1H), 7.17-7.00 (m, 1H, rotamer), 6.90 (d, J = 9.8 Hz, 1H), 6.76-6.59 (m, 1H, rotamer), 6.51 (t, J = 7.7 Hz, 2H), 6.38 (d, J = 16.8 Hz, 1H), 6.24 (t, J = 9.0 Hz, 1H), 5.81-5.70 (m, 1H, rotamer), 4.80 and 4.75 (2s, 2H, rotamer), 3.83-3.65 (m, 5H, rotamer), 2.98-2.85 (m, 2H, rotamer).

### Example 14: Preparation of 1-(5-(1-methyl-1H-imidazol-4-yl)-6-((3,4,5-trifluorophenyl)amino)-3,4-dihydroisoquinolin-2(1H)-yl)prop-2-en-1-one

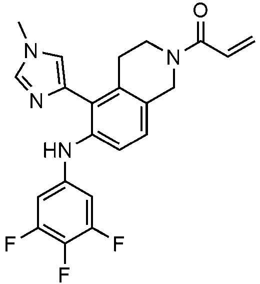

The title compound (8.9 mg, yield: 33%) was obtained in the same manner as in Example 3, except that (3,4,5-trifluorophenyl)boronic acid was used instead of (4-(trifluoromethyl)phenyl)boronic acid in step 3 of Example 3.

¹H NMR (500 MHz, CDCl₃) δ 8.45 and 8.15 (2s, 1H, rotamer), 7.60 (s, 1H), 7.27 (d, J = 8.4 Hz, 1H), 7.16-7.01 (m, 1H, rotamer), 6.91 (d, J = 9.8 Hz, 1H), 6.75-6.57 (m, 3H, rotamer), 6.38 (d, J = 16.8 Hz, 1H), 5.81-5.71 (m, 1H, rotamer), 4.79 and 4.75 (2s, 2H, rotamer), 3.84-3.64 (m, 5H, rotamer), 2.98-2.86 (m, 2H, rotamer).

### Example 15: Preparation of 1-(6-((4-chlorophenyl)amino)-5-(1-methyl-1H-imidazol-4-yl)-3,4-dihydroisoquinolin-2(1H)-yl)prop-2-en-1-one

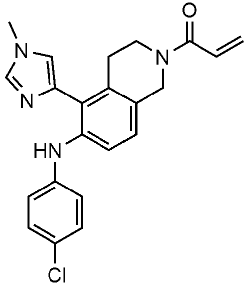

The title compound (3.1 mg, yield: 19%) was obtained in the same manner as in Example 3, except that (4-chlorophenyl)boronic acid was used instead of (4-(trifluoromethyl)phenyl)boronic acid in step 3 of Example 3.

¹H NMR (500 MHz, CDCl₃) δ 8.12 and 7.83 (2s, 1H, rotamer), 7.64 (s, 1H), 7.26 (d, J = 8.3 Hz, 1H), 7.17 (d, J = 8.7 Hz, 1H), 7.08 (d, J = 8.2 Hz, 1H), 7.00 (t, J = 7.2 Hz, 2H), 6.91 (d, J = 5.3 Hz, 2H), 6.75-6.58 (m, 1H, rotamer), 6.38 (d, J = 16.8 Hz, 1H), 5.81-5.68 (m, 1H, rotamer), 4.78 and 4.73 (2s, 2H, rotamer), 3.86-3.64 (m, 5H, rotamer), 2.99-2.80 (m, 2H, rotamer).

### Example 16: Preparation of 1-(6-((4-bromophenyl)amino)-5-(1-methyl-1H-imidazol-4-yl)-3,4-dihydroisoquinolin-2(1H)-yl)prop-2-en-1-one

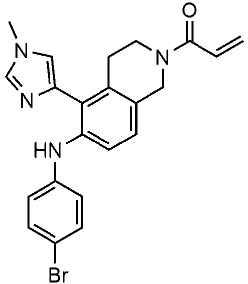

The title compound (6.9 mg, yield: 48%) was obtained in the same manner as in Example 3, except that (4-bromophenyl)boronic acid was used instead of (4-(trifluoromethyl)phenyl)boronic acid in step 3 of Example 3.

¹H NMR (500 MHz, CDCl₃) δ 8.16 and 7.85 (2s, 1H, rotamer), 7.62 (s, 1H), 7.37-7.24 (m, 3H, rotamer), 7.11-6.87 (m, 4H, rotamer), 6.78-6.58 (m, 1H, rotamer), 6.37 (d, J = 16.8 Hz, 1H), 5.81-5.69 (m, 1H, rotamer), 4.78 and 4.73 (2s, 2H, rotamer), 3.86-3.62 (m, 5H, rotamer), 2.98-2.82 (m, 2H, rotamer).

### Example 17: Preparation of 1-(6-((3,5-difluoro-4-(trifluoromethyl)phenyl)amino)-5-(1-methyl-1H-imidazol-4-yl)-3,4-dihydroisoquinolin-2(1H)-yl)prop-2-en-1-one

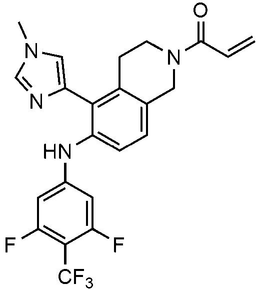

The title compound (7.9 mg, yield: 30%) was obtained in the same manner as in Example 3, except that (3,5-difluoro-4-(trifluoromethyl)phenyl)boronic acid was used instead of (4-(trifluoromethyl)phenyl)boronic acid in step 3 of Example 3.

¹H NMR (500 MHz, CDCl₃) δ 9.04 and 8.73 (2s, 1H, rotamer), 7.67 (s, 1H), 7.36 (d, J = 8.2 Hz, 1H), 7.22-7.08 (m, 1H, rotamer), 6.97-6.87 (m, 1H, rotamer), 6.75-6.59 (m, 1H, rotamer), 6.49 (dd, J = 11.6, 5.0 Hz, 2H), 6.39 (d, J = 16.6 Hz, 1H), 5.85-5.72 (m, 1H, rotamer), 4.83 and 4.79 (2s, 2H, rotamer), 3.89-3.65 (m, 5H, rotamer), 3.00-2.86 (m, 2H, rotamer).

### Example 18: Preparation of 1-(5-(1-methyl-1H-imidazol-4-yl)-6-((3,4,5-trifluorophenyl)amino)isoindolin-2-yl)prop-2-en-1-one

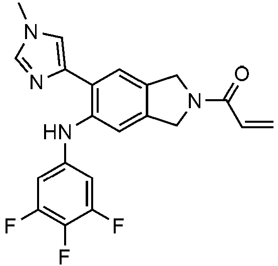

The title compound (1.1 mg, yield: 8%) was obtained in the same manner as in Example 1, except that (3,4,5-trifluorophenyl)boronic acid was used instead of (4-(trifluoromethyl)phenyl)boronic acid in step 3 of Example 1.

¹H NMR (500 MHz, CDCl₃) δ 9.65 (brs, 1H), 7.84 (s, 1H), 7.43 (s, 2H), 7.23 and 7.19 (2s, 1H, rotamer), 6.78-6.65 (m, 2H, rotamer), 6.62-6.44 (m, 2H, rotamer), 5.84-5.75 (m, 1H, rotamer), 5.02-4.84 (m, 4H, rotamer), 3.85 (s, 3H).

### Example 19: Preparation of 1-(5-((4-chlorophenyl)amino)-6-(1-methyl-1H-imidazol-4-yl)isoindolin-2-yl)prop-2-en-1-one

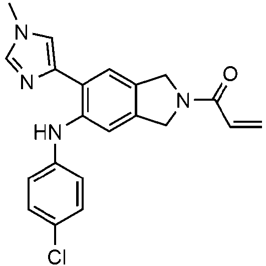

The title compound (5.0 mg, yield: 35%) was obtained in the same manner as in Example 1, except that (4-chlorophenyl)boronic acid was used instead of (4-(trifluoromethyl)phenyl)boronic acid in step 3 of Example 1.

¹H NMR (500 MHz, CDCl₃) δ 9.75 and 9.59 (2s, 1H, rotamer), 7.58 (d, J = 8.1 Hz, 1H), 7.41 and 7.38 (2s, 1H, rotamer), 7.26-7.16 (m, 4H, rotamer), 7.14 (d, J = 6.8 Hz, 2H), 6.62-6.46 (m, 2H, rotamer), 5.78 (d, J = 10.0 Hz, 1H), 4.96-4.77 (m, 4H, rotamer), 3.79 (s, 3H).

### Example 20: Preparation of 1-(5-(1-methyl-1H-imidazol-4-yl)-6-((3-(trifluoromethyl)phenyl)amino)-3,4-dihydroisoquinolin-2(1H)-yl)prop-2-en-1-one

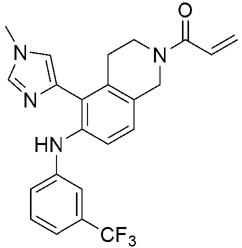

The title compound (8.6 mg, yield: 20%) was obtained in the same manner as in Example 3, except that (3-(trifluoromethyl)phenyl)boronic acid was used instead of (4-(trifluoromethyl)phenyl)boronic acid in step 3 of Example 3.

¹H NMR (500 MHz, CDCl₃) δ 8.40 and 8.08 (2s, 1H, rotamer), 7.62 (s, 1H), 7.39-7.31 (m, 2H), 7.26-7.19 (m, 2H), 7.16 - 7.01 (m, 2H), 6.92 (d, J = 8.5 Hz, 1H), 6.83 - 6.54 (m, 1H), 6.38 (d, J = 16.7 Hz, 1H), 5.76 (dd, J = 19.3, 10.5 Hz, 1H), 4.80 and 4.76 (2s, 2H, rotamer), 3.91-3.57 (m, 5H), 2.95-2.91 (m, 2H).

### Example 21: Preparation of 1-(6-((3,5-dichlorophenyl)amino)-5-(1-methyl-1H-imidazol-4-yl)-3,4-dihydroisoquinolin-2(1H)-yl)prop-2-en-1-one

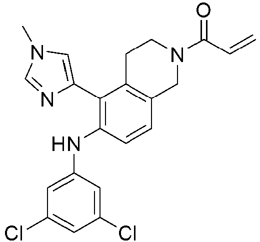

The title compound (15.0 mg, yield: 35%) was obtained in the same manner as in Example 3, except that (3,5-dichlorophenyl)boronic acid was used instead of (4-(trifluoromethyl)phenyl)boronic acid in step 3 of Example 3.

¹H NMR (500 MHz, DMSO-d₆) δ 8.68 and 8.57 (2s, 1H, rotamer), 7.75 (s, 1H), 7.25-7.20 (m, 3H), 7.00-6.72 (m, 4H), 6.22-6.12 (m, 1H), 5.80-5.65 (m, 1H), 4.80 and 4.69 (2s, 2H, rotamer), 3.69 (s, 3H), 2.85-2.80 (m, 2H), two protons were missing due to overlapping.

### Example 22: Preparation of 1-(6-((3-chloro-5-(trifluoromethyl)phenyl)amino)-5-(1-methyl-1H-imidazol-4-yl)-3,4-dihydroisoquinolin-2(1H)-yl)prop-2-en-1-one

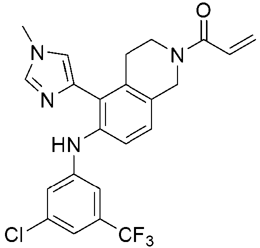

The title compound (15.9 mg, yield: 34%) was obtained in the same manner as in Example 3, except that (3-chloro-5-(trifluoromethyl)phenyl)boronic acid was used instead of (4-(trifluoromethyl)phenyl)boronic acid in step 3 of Example 3.

¹H NMR (500 MHz, CDCl₃) δ 8.71 and 8.39 (2s, 1H, rotamer), 7.61 (s, 1H), 7.33 (d, *J* = 8.3 Hz, 1H), 7.18-7.04 (m, 3H), 7.01 (s, 1H), 6.91 (d, *J* = 11.3 Hz, 1H), 6.67 (ddd, *J* = 40.9, 16.8, 10.5 Hz, 1H), 6.38 (d, *J* = 16.7 Hz, 1H), 6.00-5.51 (m, 1H), 4.81and 4.77 (2s, 2H, rotamer), 3.99-3.56 (m, 5H), 2.97-2.90 (m, 2H).

### Example 23: Preparation of 1-(6-((3,5-bis(trifluoromethyl)phenyl)amino)-5-(1-methyl-1H-imidazol-4-yl)-3,4-dihydroisoquinolin-2(1H)-yl)prop-2-en-1-one

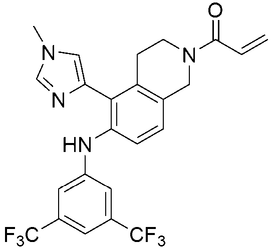

The title compound (10.9 mg, yield: 22%) was obtained in the same manner as in Example 3, except that (3,5-bis(trifluoromethyl)phenyl)boronic acid was used instead of (4-(trifluoromethyl)phenyl)boronic acid in step 3 of Example 3.

¹H NMR (500 MHz, CDCl₃) δ 8.90 and 8.58 (2s, 1H, rotamer), 7.63 (s, 1H), 7.39 (d, *J* = 7.9 Hz, 2H), 7.34 (d, *J* = 8.3 Hz, 1H), 7.25 (s, 1H), 7.13 (dd, *J* = 44.8, 8.3 Hz, 1H), 6.93 (d, *J* = 12.4 Hz, 1H), 6.68 (ddd, *J* = 39.7, 16.8, 10.5 Hz, 1H), 6.39 (d, *J* = 16.7 Hz, 1H), 5.77 (dd, *J* = 20.6, 10.5 Hz, 1H), 4.82 and 4.78 (2s, 2H, rotamer), 3.97-3.54 (m, 5H), 2.99-2.92 (m, 2H).

### Example 24: Preparation of 1-(5-(1-ethyl-1H-imidazol-4-yl)-6-((4-(trifluoromethyl)phenyl)amino)-3,4-dihydroisoquinolin-2(1H)-yl)prop-2-en-1-one

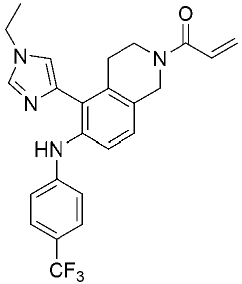

The title compound (9.1 mg, yield: 20%) was obtained in the same manner as in Example 1, except that 1-ethyl-4-(tributylstannyl)-1H-imidazole was used instead of 1-methyl-4-(tributylstannyl)-1H-imidazole in step 2 of Example 1.

¹H NMR (500 MHz, CDCl₃) δ 8.68 and 8.36 (2s, 1H, rotamer), 7.65 (d, *J* = 2.8 Hz, 1H), 7.41 (dd, *J* = 20.9, 8.4 Hz, 3H), 7.19 - 7.01 (m, 4H), 6.94 (d, *J* = 12.2 Hz, 1H), 6.68 (ddd, *J* = 37.6, 16.8, 10.5 Hz, 1H), 6.38 (dd, *J* = 16.8, 1.9 Hz, 1H), 5.76 (ddd, *J* = 19.6, 10.5, 1.9 Hz, 1H), 4.80 - 4.76 (2s, 2H, rotamer), 4.07 (q, *J* = 7.4 Hz, 2H), 3.80 (t, *J* = 5.9 Hz, 1H, rotamer), 3.70 (t, *J* = 5.9 Hz, 1H, rotamer), 2.97 - 2.92 (m, 2H), 1.53 (t, *J* = 7.4 Hz, 3H).

### Experimental Example 1: Activity inhibition test of transcription factor TEAD

The transcription factor TEAD activity of the compounds prepared in the Examples was measured by the ONE-Glo^{™} Luciferase assay (Promega, Catalog #E6110) method using the Hippo Pathway TEAD reporter-MCF7 recombinant cell line (BPS Bioscience, Catalog #60618).

Specifically, the TEAD Reporter-MCF7 cell line contains the firefly luciferase gene whose expression is regulated under the control of TEAD response elements in the MCF7, which is a human breast cancer cell line. In the cell line, a non-phosphorylated YAP/TAZ exists in the nucleus in a non-stressed condition, which continuously induces expression of the luciferase reporter.

TEAD Reporter-MCF7 cells were prepared in 100 µL of cell culture medium (MEM medium, 10% FBS, 1% P/S, 400 µg/mL Geneticin, 1% N-ethyl acetate A, 1 mM NA pyruvate, 10 µg/mL Insulin) in a white clear bottom 96-well microplate. The prepared plate was incubated in a 5% CO₂ incubator at 37°C for 24 hours, and then the previously prepared compounds of Examples were finally treated at concentrations of 0.0005, 0.005, 0.05, 0.5 and 5 µM, which were repeated three times for all treatment solutions. The plates treated with the compounds of Examples were incubated in a 5% CO₂ incubator at 37°C for 24 hours. 100 µL/well of the substrate aqueous solution containing luciferin was added to a white clear-bottom 96 well microplate, thereby initiating an enzyme reaction. The reaction was performed at room temperature for 5 minutes, and the luminescence (integration time 1000 ms) was measured using a Flexstation3 multi-mode microplate reader. In accordance with the instructions for the ONE-Glo^{™} Luciferase Assay, the luciferase enzyme activity, which represents the activity of the transcription factor TEAD, was measured by a chemiluminescence method, and the inhibitory activity of the compound according to the present disclosure was calculated. The results of each compound were analyzed using Microsoft Excel, and the IC₅₀ values were calculated by GraphPad Prism software. The results are shown in Table 1 below.

### Experimental Example 2: Cell growth inhibition test

The cell growth for the compounds prepared in Examples was measured by CellTiter-Glo^{®} Luminescednt Cell Viability (Promega, Catalog #G7571) method using NCI-H226 (addexbio), NCI-H28 (Korea Cell Line Bank) cell lines. This evaluation method is a method in which when luciferase enzyme is activated by ATP flowing out from living cells, it reacts with a luciferin substrate, and the enzyme activity at this time is measured to confirm cell viability.

Specifically, NCI-H226, and NCI-H28 cells were prepared in 100 µL of cell culture medium (RPMI medium, 10% FBS, 1% P/S, 4.5 g/L D-Glucose, 2.383 g/L HEPES Buffer, L-Glutamine, 1.5 g/L Sodium Bicarbonate, 110 mg/L Sodium pyruvate) in a white clear bottom 96-well microplate. The prepared plate was incubated in a 5% CO₂ incubator at 37°C for 24 hours, and then the previously prepared compounds of Examples were finally treated at concentrations of 0.0005, 0.005, 0.05, 0.5 and 5 µM, which was repeated three times for all treatment solutions. The plates treated with the compounds of Examples were incubated in a 5% CO₂ incubator at 37°C. 100 µL/well of the substrate aqueous solution containing luciferin was added to a white clear-bottom 96 well microplate, thereby initiating an enzyme reaction. The reaction was performed at room temperature in the dark for 10 minutes, and the luminescence was measured using a Flexstation3 multi-mode microplate reader. In accordance with the instructions for the CellTiter-Glo^{™} Luminescednt Cell Viability, the luciferase enzyme activity, which represents the amount of ATP, was measured by a chemiluminescence method, and the inhibitory activity of the compounds according to the present disclosure was calculated. The results of each compound were analyzed using Microsoft Excel, and the IC₅₀ values were calculated using GraphPad Prism software. The results are shown in Table 1 below.

**[Table 1]**

| Example No. | TEAD IC50 (µM) | growth IC₅₀ (µM) H226 | growth IC₅₀ (µM) H28 |
|---|---|---|---|
| 1 | 0.0427 | 0.0212 | 3.0000 |
| 2 | 1.4600 | 0.0898 | 5.2000 |
| 3 | 0.0255 | 0.0098 | 0.9512 |
| 4 | 4.8400 | 1.35 | 10.0000 |
| 5 | 0.6023 | 0.1192 | 2.0000 |
| 6 | 1.5600 | 0.9231 | 6.6000 |
| 7 | 0.0143 | 0.0378 | 2.1800 |
| 8 | 9.3470 | 1.53 | 10.0000 |
| 9 | 0.0342 | 0.036 | 0.7783 |
| 10 | 0.0496 | 0.1003 | 1.5000 |
| 11 | 1.8890 | N/D | N/D |
| 12 | >10 | N/D | N/D |
| 13 | 0.02804 | 0.0553 | 2.0000 |
| 14 | 0.006777 | 0.0166 | 1.8000 |
| 15 | 0.0059 | 0.0309 | 2.4000 |
| 16 | 0.0081 | 0.0352 | 0.6358 |
| 17 | 0.0086 | 0.0096 | 3.3000 |
| 18 | 0.2394 | N/D | N/D |
| 19 | 0.1181 | N/D | N/D |
| 20 | 0.0450 | 0.139 | 1.1200 |
| 21 | 0.0902 | 0.095 | >5 |
| 22 | 0.2424 | N/D | N/D |
| 23 | >5 | N/D | N/D |
| 24 | 0.0154 | 0.080 | >5 |

### Experimental Example 3: CTGF, CYR61 Analysis Test

NCI-H226 (AddexBio) human mesothelioma cells were cultured in a 6-well cell culture plate at a density of 5x10⁵ cells per well. After a stabilization process for 24 hours, Examples 1 and 17 was treated at a concentration of 500 nM, and cells treated with the same amount of DMSO were set as a control. 24 hours after treatment, cells were collected to secure RNA for target gene expression analysis.

RNA was extracted from cells using a TRIzol solution (Ambion). Once cell lysis and digestion were completed, chloroform was added to each sample and the homogenate was separated into aqueous and organic phases by centrifugation.

The expression of ctgf (connective tissue growth factor) and cyr61 (Cysteine-rich angiogenic inducer 61), which are genes regulated by YAP-TEAD, and gapdh(Glyceraldehyde 3-phosphate dehydrogenase), which is a housekeeping gene, were quantified by qRT-PCR analysis using the Maxima SYBR Green/Fluorescein qPCR Master Mix and the primers for each gene. The ctgf, cyr61 and gapdh cycle threshold (Ct) values of cell cDNA samples were determined, and ctgf, cyr61 expression was normalized to gapdh.

The expression of each gene in the experimental group treated with Examples 1 and 17 was normalized relative to the control treated with DMSO. FIG. 1 shows the expression regulatory properties of two TEAD target genes in Examples 1 and 17 using real-time PCR.

## Claims

1. A compound represented by the following Chemical Formula 1, or a pharmaceutically acceptable salt thereof: 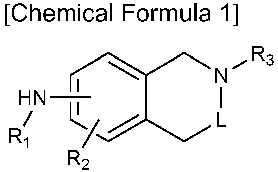
in Chemical Formula 1,
L is a single bond, or a C₁₋₃ linear alkylene,
R₁ is a C₆₋₁₀ aryl, or a 5- or 6-membered heterocycle containing 1 to 4 heteroatoms each independently selected from the group consisting of N, O and S,
wherein the R₁ is unsubstituted, or substituted with 1 to 3 substituents each independently selected from the group consisting of halogen, a C₁₋₄ alkyl, a C₁₋₄ thioalkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, a C₃₋₆ cycloalkyl, amino, nitro, cyano, a (C₁₋₄ alkyl)amino, and a di(C₁₋₄ alkyl)amino,
R₂ is a C₆₋₁₀ aryl, or a 5- or 6-membered heterocycle containing 1 to 4 heteroatoms each independently selected from the group consisting of N, O and S,
wherein the R₂ is unsubstituted, or substituted with halogen, a C₁₋₄ alkyl, a C₁₋₄ thioalkyl, a C₁₋₄ haloalkyl, a C₁₋₄ alkoxy, a C₁₋₄ thioalkoxy, a C₁₋₄ haloalkoxy, a C₂₋₄ alkenyl, a C₂₋₄ alkynyl, a C₃₋₆ cycloalkyl, amino, nitro, cyano, a (C₁₋₄ alkyl)amino, and a di(C₁₋₄ alkyl)amino, or is fused with a C₃₋₆ cycloalkyl ring,
R₃ is -CO-R₄, -SO₂-NH-R₄, or -CH₂-R₄, and
R₄ is hydrogen, a C₁₋₄ alkyl, a C₂₋₄ alkynyl, or cyano.

2. The compound or the pharmaceutically acceptable salt thereof according to claim 1, wherein:
L is a single bond, or methylene.

3. The compound or the pharmaceutically acceptable salt thereof according to claim 1, wherein:
the Chemical Formula 1 is represented by any one of the following Chemical Formulas 1-1 to 1-4: 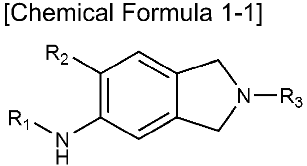 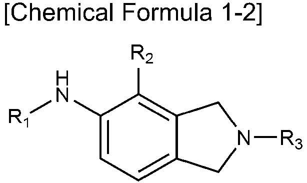 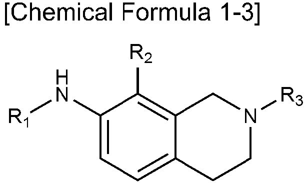 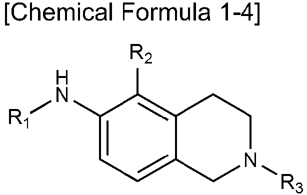
wherein in Chemical Formulas 1-1 to 1-4,
R₁, R₂ and R₃ are as defined in claim 1.

4. The compound or the pharmaceutically acceptable salt thereof according to claim 1, wherein:
R₁ is phenyl, pyridinyl, pyrimidinyl, imidazolyl, pyrazolyl, triazolyl, tetrazolyl, oxadiazolyl, pyrrolidinyl, imidazolyl, pyrazolyl, oxazolyl, isoxazolyl, thiazolyl, or isothiazolyl,
wherein the R₁ is unsubstituted, or substituted with halogen, a C₁₋₄ alkyl, a C₁₋₄ thioalkyl, a C₁₋₄ haloalkyl, a C₁₋₄ alkoxy, a C₁₋₄ haloalkoxy, amino, nitro, cyano, a (C₁₋₄ alkyl)amino, or a di(C₁₋₄ alkyl)amino.

5. The compound or the pharmaceutically acceptable salt thereof according to claim 1, wherein:
R₁ is phenyl,
wherein the R₁ is unsubstituted, or substituted with 1 to 3 substituents each independently selected from the group consisting of fluoro, chloro, methyl and trifluoromethyl.

6. The compound or the pharmaceutically acceptable salt thereof according to claim 1, wherein:
R₂ is phenyl, pyridinyl, pyrimidinyl, imidazolyl, pyrazolyl, triazolyl, tetrazolyl, oxadiazolyl, pyrrolidinyl, imidazolyl, pyrazolyl, oxazolyl, isoxazolyl, thiazolyl, or isothiazolyl,
wherein the R₂ is unsubstituted, or substituted with halogen, a C₁₋₄ alkyl, a C₁₋₄ thioalkyl, a C₁₋₄ haloalkyl, a C₁₋₄ alkoxy, a C₁₋₄ haloalkoxy, amino, nitro, cyano, a (C₁₋₄ alkyl)amino, or di(C₁₋₄ alkyl)amino, or is fused with a C₃₋₆ cycloalkyl ring.

7. The compound or the pharmaceutically acceptable salt thereof according to claim 1, wherein:
R₂ is imidazolyl,
wherein the R₂ is substituted with methyl, or ethyl, or is fused with a cyclopentane ring.

8. The compound or the pharmaceutically acceptable salt thereof according to claim 1, wherein:
R₄ is hydrogen, methyl, ethenyl, or cyano.

9. The compound or the pharmaceutically acceptable salt thereof according to claim 1, wherein:
R₃ is -CO-CH=CH₂, -SO₂-NH-CH₃, or -CH₂-CN.

10. The compound or the pharmaceutically acceptable salt thereof according to claim 1, wherein the compound represented by Chemical Formula 1 is any one selected from the group consisting of:
1) 1-(5-(1-methyl-1H-imidazol-4-yl)-6-((4-(trifluoromethyl)phenyl)amino)isoindolin-2-yl)prop-2-en-1-one,
2) N-methyl-5-(1-methyl-1H-imidazol-4-yl)-6-((4-(trifluoromethyl)phenyl)amino)isoindoline-2-sulfonamide,
3) 1-(5-(1-methyl-1H-imidazol-4-yl)-6-((4-(trifluoromethyl)phenyl)amino)-3,4-dihydroisoquinolin-2(1H)-yl)prop-2-en-1-one,
4) N-methyl-5-(1-methyl-1H-imidazol-4-yl)-6-((4-(trifluoromethyl)phenyl)amino)-3,4-dihydroisoquinoline-2(1H)-sulfonamide,
5) 1-(4-(1-methyl-1H-imidazol-4-yl)-5-((4-(trifluoromethyl)phenyl)amino)isoindolin-2-yl)prop-2-en-1-one,
6) N-methyl-4-(1-methyl-1H-imidazol-4-yl)-5-((4-(trifluoromethyl)phenyl)amino)isoindoline-2-sulfonamide,
7) 1-(5-(6,7-dihydro-5H-pyrrolo[1,2-a]imidazol-2-yl)-6-((4-(trifluoromethyl)phenyl)amino)-3,4-dihydroisoquinolin-2(1H)-yl)prop-2-en-1-one,
8) 2-(5-(1-methyl-1H-imidazol-4-yl)-6-((4-(trifluoromethyl)phenyl)amino)isoindolin-2-yl)acetonitrile,
9) 1-(6-((3-fluoro-4-(trifluoromethyl)phenyl)amino)-5-(1-methyl-1H-imidazol-4-yl)-3,4-dihydroisoquinolin-2(1H)-yl)prop-2-en-1-one,
10) 1-(6-((4-fluorophenyl)amino)-5-(1-methyl-1H-imidazol-4-yl)-3,4-dihydroisoquinolin-2(1H)-yl)prop-2-en-1-one,
11) 1-(5-((4-fluorophenyl)amino)-6-(1-methyl-1H-imidazol-4-yl)isoindolin-2-yl)prop-2-en-1-one,
12) 1-(7-((4-fluorophenyl)amino)-8-(1-methyl-1H-imidazol-4-yl)-3,4-dihydroisoquinolin-2(1H)-yl)prop-2-en-1-one,
13) 1-(6-((3,5-difluorophenyl)amino)-5-(1-methyl-1H-imidazol-4-yl)-3,4-dihydroisoquinolin-2(1H)-yl)prop-2-en-1-one,
14) 1-(5-(1-methyl-1H-imidazol-4-yl)-6-((3,4,5-trifluorophenyl)amino)-3,4-dihydroisoquinolin-2(1H)-yl)prop-2-en-1-one,
15) 1-(6-((4-chlorophenyl)amino)-5-(1-methyl-1H-imidazol-4-yl)-3,4-dihydroisoquinolin-2(1H)-yl)prop-2-en-1-one,
16) 1-(6-((4-bromophenyl)amino)-5-(1-methyl-1H-imidazol-4-yl)-3,4-dihydroisoquinolin-2(1H)-yl)prop-2-en-1-one,
17) 1-(6-((3,5-difluoro-4-(trifluoromethyl)phenyl)amino)-5-(1-methyl-1H-imidazol-4-yl)-3,4-dihydroisoquinolin-2(1H)-yl)prop-2-en-1-one,
18) 1-(5-(1-methyl-1H-imidazol-4-yl)-6-((3,4,5-trifluorophenyl)amino)isoindolin-2-yl)prop-2-en-1-one,
19) 1-(5-((4-chlorophenyl)amino)-6-(1-methyl-1H-imidazol-4-yl)isoindolin-2-yl)prop-2-en-1-one,
20) 1-(5-(1-methyl-1H-imidazol-4-yl)-6-((3-(trifluoromethyl)phenyl)amino)-3,4-dihydroisoquinolin-2(1H)-yl)prop-2-en-1-one,
21) 1-(6-((3,5-dichlorophenyl)amino)-5-(1-methyl-1H-imidazol-4-yl)-3,4-dihydroisoquinolin-2(1H)-yl)prop-2-en-1-one,
22) 1-(6-((3-chloro-5-(trifluoromethyl)phenyl)amino)-5-(1-methyl-1H-imidazol-4-yl)-3,4-dihydroisoquinolin-2(1H)-yl)prop-2-en-1-one,
23) 1-(6-((3,5-bis(trifluoromethyl)phenyl)amino)-5-(1-methyl-1H-imidazol-4-yl)-3,4-dihydroisoquinolin-2(1H)-yl)prop-2-en-1-one, and
24) 1-(5-(1-ethyl-1H-imidazol-4-yl)-6-((4-(trifluoromethyl)phenyl)amino)-3,4-dihydroisoquinolin-2(1H)-yl)prop-2-en-1-one.

11. A pharmaceutical composition for the prevention or treatment of cancer or tumors, comprising the compound according to any one of claims 1 to 10, or a pharmaceutically acceptable salt thereof as an active ingredient.
